(19) Europäisches Patentamt
European Patent Office
Office européen des brevets

(11) Numéro de publication: 0 036 420
B1

(12) # FASCICULE DE BREVET EUROPEEN

(45) Date de publication du fascicule du brevet: **10.08.83**

(51) Int. Cl.³: **C 07 C 103/52,** A 61 K 37/02

(21) Numéro de dépôt: **80901851.8**

(22) Date de dépôt: **01.10.80**

(86) Numéro de dépôt international: **PCT/FR 80/00142**

(87) Numéro de publication internationale: **WO 81/00847 (02.04.81** Gazette **81/8)**

(54) **PEPTIDES AYANT DES POPRIETES IMMUNOSTIMULANTES ET COMPOSITIONS PHARMACEUTIQUES LES CONTENANT.**

(30) Priorité: **01.10.79 FR 7924448**

(43) Date de publication de la demande: **30.09.81 Bulletin 81/39**

(45) Mention de la délivrance du brevet: **10.08.83 Bulletin 83/32**

(84) Etats contractants désignés: **CH DE GB LI NL**

(56) Documents cités:
**Chemical Abstracts, vol. 81, no. 11,** *publié le 16 septembre 1974* **(Columbus, Ohio, US), R.J. Beuhler et al.: "Proton transfer mass spectrometry of peptides. Rapid heating technique for underivatized peptides containing arginine", voir page 508, colonne 2, l'abrégé no. 63947g**
**Chemical Abstracts, vol. 83, no. 1, publié le 7 juillet 1975 (Columbus, Ohio, US), L. Balaspiri et al.: "Preparation of fragments of bradykinin analogs containing optically active pipecolic acid", voir pages 916,917 l'abrégé no. 10811t**
**G.R. PETIT: "Synthetic Peptides", vol. 1, Edition 4, Van Nostrand Reinhold Comp. (New York, US), voir page 262, 4e-3e lignes d'en bas**
**Nature, vol. 187, no. 4739, publié le 27 août 1960, Edit. McMillan Ltd. (Londres, GB), E.D. Nicolaides et H.A. de**

(73) Titulaire: **INSTITUT PASTEUR, 25-28, rue du Docteur Roux, F-75724 Paris Cedex 15 (FR)**

(72) Inventeur: **FAUVE, Robert, 18, rue de la Justice, F-92310 Sèvres (FR)**

(74) Mandataire: **Gutmann, Ernest, Cabinet Plasseraud 84, rue d'Amsterdam, F-75009 Paris (FR)**

(56) Documents cités:
**Wald: "Synthesis and biological properties of L-arginyl-L-propyl-L-propyl-glycyl-L-phenylalanyl-L-seryl-L-phenylalanyl-L-arginine, an octapeptide related to bradykinin", voir pages 773-774**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

Peptides ayant des propriétés immunostimulantes et compositions pharmaceutiques les contenant

L'invention est relative à de nouveaux peptides ayant des propriétés biologiques, notamment immunostimulantes. Elle concerne plus particulièrement, à titre de produits industriels nouveaux, des peptides de petite taille comportant - si l'on fait abstraction de l'isomérie optique possible de leurs différents résidus aminoacyle - une séquence peptidique (ci-après dite «séquence peptidique commune») en commun avec la bradykinine et au moins un résidu aminoacyle distinct de celui auquel est rattachée l'extrémité N-terminale de ladite «séquence peptidique commune» dans la bradykinine elle-même. En particulier, un peptide selon l'invention, qui comporte au plus sept résidus aminoacyle, et contient une séquence prolyl → prolyl → glycyle, le groupe carboxylique du résidu glycyle étant, le cas échéant, engagé dans une liaison peptidique avec un autre résidu aminoacyle, est caractérisé en ce que le groupe aminé du premier résidu prolyle est engagé dans une liaison peptidique avec un résidu aminoacyle différent de l'arginyle, de préférence du type lysyle ou histidyle.

La formule de la bradykinine peut être représentée comme suit:

Arginyl → prolyl → prolyl → glycyl → phénylalanyl → séryl → prolyl → phénylalanyl → arginine

(le sens des flèches indique la direction de la liaison peptidique: de l'extrémité N-terminale vers l'extrémité C-terminale). Cette nomenclature sera également adoptée dans ce qui suit en ce qui concerne la représentation des formules des peptides selon l'invention.

Des peptides plus petits (parmi lesquels le pentapeptide arginyl → prolyl → prolyl → glycyl → phénylalanine) correspondant à des séquences de la bradykinine ont été décrits dans la littérature. Au titre de l'état de la technique, on peut se référer aux documents suivants:

- Journal of the American Chemical Society, 1974, 96(12), pages 3990-9, R.J. Beuhler et al.: «Proton transfer mass spectrometry of peptides. Rapid heating technique for underivatized peptides containing arginine»,
- Acta Phys. Chem. 1974, 20(4), pages 465-470, L. Balaspiri et al.: «Preparation of fragments of bradykinin analogs containing optically active pipecolic acid»,
- George R. Pettit: «Synthetic peptides», Van Nostrand Reinhold Company, New-York (US), pages 262, 460,
- Nature, vol. 187, no. 4739, 27 août 1960, Mc Millan Ltd., Londres (GB), E.D. Nicolaides et al.: «Synthesis and biological properties of L-Arginyl-L-Prolyl-L-Prolyl-Glycyl-L-Phenylalanyl-L-Seryl-L-Phenylalanyl-L-Arginine, an Octapeptide related to Bradykinin», pages 773-774,
- Canadian Journal of Physiology and Pharmacology, 1977, vol. 55, pages 855-867, D. Regoli et al.

Dans la mesure où certains de ces articles ont fait référence à des essais biologiques sur des peptides plus petits que la bradykinine, il a été constaté, surtout en ce qui concerne des peptides comprenant au plus sept résidus aminoacyle, qu'ils étaitent tout à fait dénués des activités antérieurement connues de la bradykinine. La découverte que certains d'entre eux possèdent d'importantes propriétés immunostimulantes n'en a été que plus surprenante.

L'invention concerne donc également les compositions pharmaceutiques, applicables au traitement des maladies impliquant un mécanisme d'activation des macrophages et contenant, en association avec un véhicule physiologiquement acceptable, un peptide comprenant au plus sept résidus aminoacyle et comportant la susdite «séquence peptidique commune» dont l'extrémité N-terminale est reliée soit à l'arginyle (correspondant au résidu aminoacyle que contient la bradykinine en l'endroit correspondant de sa chaîne peptidique), soit à un résidu aminoacyle distinct.

Au titre des produits industriels nouveaux, l'invention concerne donc plus particulièrement les nouveaux peptides comportant au plus sept aminoacides et caractérisés en ce qu'ils contiennent une séquence prolyl → prolyl → glycyl, le groupe carboxylique du résidu glycyle étant, le cas échéant, engagé dans une liaison peptidique avec un autre résidu aminoacyle, et en ce que le groupe aminé du premier résidu prolyle de la susdite séquence prolyl → prolyl → glycyl est engagé dans une liaison peptidique avec un résidu lysyle ou histidyle.

Au titre des peptides actifs entrant dans les compositions pharmaceutiques préférées de l'invention, on mentionnera ceux qui comportent au plus sept aminoacides et qui contiennent une séquence prolyl → prolyl → glycyl, le groupe carboxylique du résidu glycyle étant, le cas échéant, engagé dans une liaison peptidique avec un autre résidu aminoacyle.

Avantageusement ces peptides contiennent une séquence prolyl → prolyl → glycyl → phénylalanyle.

L'invention concerne plus particulièrement ceux de ces peptides dans lesquels le susdit résidu phénylalanyle porte l'extrémité C-terminale du peptide.

L'invention concerne plus particulièrement encore:

- au titre des produits industriels nouveaux préférés, des peptides tels que ci-dessus définis dans lesquels le groupe aminé du premier résidu prolyle de la susdite séquence prolyl → prolyl → glycyle est engagé dans une liaison peptidique avec un résidu lysyle ou histidyle, et
- au titre des compositions pharmaceutiques préférées, celles dont le principe actif est constitué par l'un des produits industriels nouveaux préférés susdits ou un peptide dans lequel le groupe aminé du premier résidu prolyle de la susdite séquence prolyl → prolyl → glycyle est engagé dans une liaison peptidique avec un résidu arginyle.

Les composés préférés de la série qui précède sont constitués par des pentapeptides dont les représentants préférés peuvent être caractérisés par les formules suivantes:
Arginyl → prolyl → prolyl → glycyl → phénylalanine, Lysyl → prolyl → prolyl → glycyl → phénylalanine, Histidyl → prolyl → prolyl → glycyl → phénylalanine.

Le premier de ces pentapeptides est donc constitué des mêmes résidus aminoacyle que ceux de la séquence des cinq premiers aminoacides de la bradykinine, d'où la désignation «1-5» utilisée quelquefois dans ce qui suit pour le désigner.

Les deux autres pentapeptides diffèrent du premier par la nature du premier résidu aminoacyle (C-terminal), la lysine pour le second, la histidine pour le troisième, d'où les abréviations «L5» et «H5» qui seront utilisées pour les désigner quelquefois dans ce qui suit.

Le cas échéant, les peptides selon l'invention tels qu'ils ont été définis ci-dessus, comportent un ou deux résidus aminoacyle supplémentaires, notamment tels que lysyle et/ou méthionyle, reliés notamment aux résidus aminoacyle N-terminaux des susdits peptides par l'intermédiaire de la liaisons peptidiques.

Les susdits pentapeptides constituent les principes actifs préférés de l'invention. Elle s'étend cependant aussi aux peptides comportant davantage de résidus aminoacyle. A cet égard, il est intéressant de mentionner que les dérivés de la bradykinine constitués par des peptides comportant notamment la totalité de la séquence peptidique de la bradykinine et un ou deux résidus aminoacyle supplémentaires, tels que lysyle et/ou méthionyle, respectivement reliés par l'intermédiaire de liaisons peptidiques au résidu N-terminal de la bradykinine, possèdent également une activité immunostimulante.

A titre d'exemples supplémentaires préférés de principes actifs, on citera:

1°) la lysyl → prolyl → prolyl → glycyl → phénylalanyl → sérine;
2°) la lysyl → prolyl → prolyl → glycyl → phénylalanyl → séryl → proline;
3°) la histidyl → prolyl → prolyl → glycyl → phénylalanyl → sérine;
4°) la histidyl → prolyl → prolyl → glycyl → phénylalanyl → séryl → proline;
5°) l'arginyl → prolyl → prolyl → glycyl → phénylalanyl → sérine;
6°) l'arginyl → prolyl → prolyl → glycyl → phénylalanyl → séryl → proline.

Le sixième et/ou le septième résidus aminoacyle des peptides susdits peuvent, le cas échéant, être remplacés par un et/ou deux résidus aminoacyle distincts.

Les résidus aminoacyle successifs des peptides selon l'invention sont de préférence tous constitués par leurs isomères lévogyres, à l'exception des résidus glycyle qui ne sont pas susceptibles de former des isomères respectivement dextrogyres et lévogyres. Un ou plusieurs des résidus aminoacyle des peptides susdits peuvent néanmoins également être sous forme dextrogyre. Il en résulte des produits moins biodégradables.

Les peptides selon l'invention sont de préférence obtenus par synthèse peptidique, notamment selon la méthode décrite par R.B. Merrifield, dans l'article intitulé «Solid Phase Peptide Synthesis. I The Synthesis of a Tetrapeptide» (Synthèse Peptidique en Phase Solide. I La Synthèse d'un Tétrapeptide), [J. Am. Chem. Soc. 45 (1963), 2149-2154].

L'invention concerne également les dérivés fonctionnels physiologiquement acceptables de ces peptides, notamment les sels d'acides ou d'amines qu'ils peuvent former avec des acides ou bases physiologiquement acceptables, ou des dérivés de substitution du type amide ou ester, pour ce qui est de l'acide aminé C-terminal, ou dérivés du type sel d'ammonium ou amine secondaire ou tertiaire, pour ce qui est de l'acide aminé N-terminal.

D'autres caractéristiques de l'invention ressortiront encore de la description qui suit des propriétés biologiques de quelques-uns des peptides selon l'invention considérés à titre d'exemples.

Les peptides selon l'invention peuvent être synthétisés par des procédés classiques, par exemple du type de ceux répondant aux schémas de réaction suivants, en ce qui concerne les deux pentapeptides Lys-Pro-Pro-Gly-Phe et His-Pro-Pro-Gly-Phe.

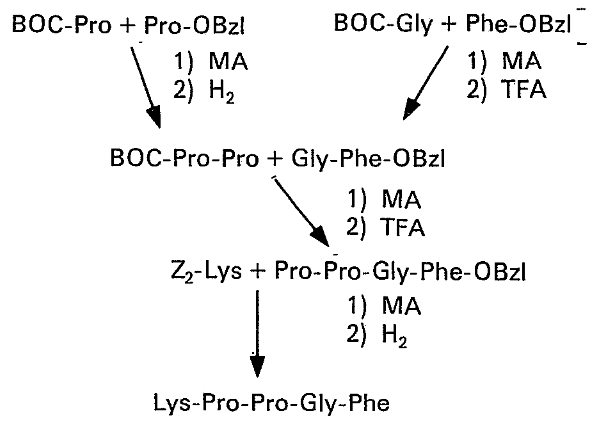


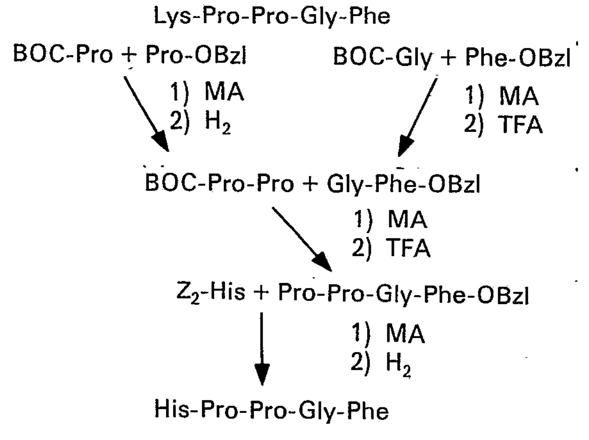

les abréviations utilisées ayant les significations suivantes:

MA : anhydrides mixtes
TFA: acide trifluoroacétique
BOC: t-butyloxycarbonyle
Z: benzyloxycarbonyle
OBzl: benzylester.

Propriétés biologiques

Les résultats obtenus avec les produits «1-5», «L5» et «H5» sont représentatifs de ceux qui peuvent être mis en évidence à l'occasion de la mise en œuvre des principes actifs de l'invention.

1. Augmentation de la survie des souris à une infection par Listeria monocytogenes après traitement par les principes actifs de l'invention.

Les souris de groupes de 11 animaux ont reçu, par voie intraveineuse, soit uniquement 0,5 ml d'une solution apyrogène de ClNa 0,15 M, soit 0,5 ml de la même solution contenant $10^{-4}$ M desdits principes actifs. Douze heures plus tard, chaque groupe est divisé en deux lots de cinq et six souris. Les souris du premier lot ont reçu, par voie intraveineuse, $1,2 \times 10^5$ Listeria monocytogenes et celles du second $0,6 \times 10^5$ Listeria monocytogenes.

72 heures plus tard on constate que la plupart des animaux traités ont survécu, ce qui manifeste un accroissement considérable de leur résistance à l'égard de Listeria monocytogenes, alors que les témoins ont tous péri.

2. Diminution de la multiplication de Listeria monocytogenes dans le foie et la rate des souris traitées par les principes actifs de l'invention.

Les souris de quatre groupes de 4 animaux ont été traitées dans les mêmes conditions que précédemment. Douze heures plus tard, les souris sont toutes inoculées, par voie intraveineuse avec $1,3 \times 10^5$ Listeria monocytogenes.

Le tableau ci-après résume les résultats obtenus. A nouveau, les souris traitées par les principes actifs présentent, dans leur rate et surtout dans leur foie, un nombre de Listeria jusqu'à 5000 fois moins important que dans le foie des animaux témoins.

| Traitement des animaux | Nombre de Listeria monocytogenes dans la rate des souris 48 h après infection | |
|---|---|---|
| ClNa 0,15 M | $7 \times 10^7 \pm 2,4$ | |
| 1-5 | $3,1 \times 10^7 \pm 1$ | $p < 0,05$ |
| L5 | $1,8 \times 10^7 \pm 0,28$ | $p < 0,0125$ |
| H5 | $2,4 \times 10^7 \pm 1,3$ | $p < 0,05$ |

3. Accélération de l'épuration sanguine de Salmonella typhimurium chez les souris traitées par les principes actifs de l'invention.

L'augmentation du pouvoir bactéricide des macrophages s'accompagne toujours d'une augmentation de leur capacité d'ingestion. On a cherché à mesurer celle-ci avec Salmonella typhimurium, qui nécessite pour sa phagocytose des anticorps opsonisants ou une stimulation importante des macrophages hépatiques, ce qui n'est pas le cas pour Listeria monocytogenes. Pour cela, les souris ont été traitées dans les mêmes conditions que dans l'expérience précédente, mais ont été inoculées avec $2 \times 10^7$ Salmonella typhimurium par voie intraveineuse.

Les résultats rapportés dans le tableau ci-après montrent que la bactériémie est de 5 à 20 fois moins élevée que chez les animaux témoins.

| Traitement des animaux | Nombre de Salmonella typhimurium par ml de sang 2 h après infection | |
|---|---|---|
| ClNa 0,15 M | $1,20 \times 10^6 \pm 0,28$ | |
| 1-5 | $3,05 \times 10^5 \pm 1,6$ | $p < 0,01$ |
| L5 | $9,35 \times 10^4 \pm 7,9$ | $p < 0,0025$ |
| H5 | $7,15 \times 10^4 \pm 0,78$ | $p < 0,0025$ |

Ces résultats montrent que l'injection préalable des principes actifs à des souris entraîne une augmentation de résistance de ces animaux envers deux bactéries très virulentes pour la souris: Listeria monocytogenes et Salmonella typhimurium.

Les tests biologiques qui ont été mis en œuvre dans ce qui précède sont, du fait de leur sévérité et de l'analogie reconnue des mécanismes infectieux chez l'homme et la souris, extrapolables à l'homme avec un degré de présomption reconnu comme particulièrement significatif par les spécialistes.

4. Augmentation de la résistance des souris traitées par les peptides selon l'invention envers une des tumeurs les plus malignes de la souris: le carcinome de Lewis (3LL).

Cette tumeur est entretenue au laboratoire ainsi qu'il l'a été décrit (Fauvre, R.M. et Hevin, B., Ann. Immunol. (Inst. Pasteur), 1977, 128C, 923-928). Des cellules 3LL sont cultivées dans du milieu de Eagle, dont la composition est indiquée ci-après, supplémenté par 15% de sérum fœtal de veau. Le milieu de Eagle, décrit par John Paul «Cell and Tissue Cultures», Ed. Williams and Wilkins Company Baltimore, E.U.A., 1961, p. 95, a la

composition suivante (tous les acides aminés indiqués ci-après sont des L-amino-acides):

| | |
|---|---|
| arginine | 17,4 mg/l |
| cystine | 6 |
| cystidine | 3,2 |
| isoleucine | 26,2 |
| leucine | 13,1 |
| lycine | 18,2 |
| méthionine | 7,5 |
| phénylalanine | 8,3 |
| tréonine | 11,9 |
| tryptophane | 2 |
| thyrosine | 18 |
| valine | 11,7 |
| glutamine | 146 |
| choline | 1 |
| acide nicotinique | 1 |
| acide pentothénique | 1 |
| pyridoxal | 1 |
| riboflavine | 0,1 |
| thiamine | 1 |
| i-inositol | 1 |
| biotine | 1 |
| acide valique | 1 |
| glucose | 2000 |
| NaCl | 8000 |
| KCl | 400 |
| $CaCl_2$ | 140 |
| $MgSO_4, 7H_2O$ | 100 |
| $MgCl_2, 6H_2O$ | 100 |
| $Na_2HPO_4, 2H_2O$ | 60 |
| $KH_2PO_4$ | 60 |
| $NaHCO_3$ | 350 |
| rouge de phénol | 20 |
| pénicilline | 0,050 |

Après lavage et remise en suspension dans du milieu de Eagle sans sérum, ces cellules 3LL sont injectées à raison de $10^5$ cellules sous un volume de 50 µl dans le coussinet plantaire de souris C57B1/6. La différence entre les épaisseurs de la patte où se développe la tumeur et de la patte contralatérale permet d'apprécier le développement de la tumeur.

Dans tous les cas, les produits testés ont été injectés 24 heures après l'inoculation des cellules malignes et, 9 jours plus tard, après avoir mesuré l'épaisseur des pattes, la patte portant la tumeur est amputée et, 11 jours plus tard, soit 20 jours après l'inoculation des tumeurs, les animaux sont autopsiés et le nombre de nodules métastasiques pulmonaires comptés. Le tableau suivant résume les résultats obtenus.

| Traitement des animaux (10 souris par lot) | Développement de la tumeur | Nombre de métastases pulmonaires |
|---|---|---|
| ClNa 0,15 M | 326±34 | 5,3±2,7 |
| 1-5 | 284±33 p <0,0125 | 0,7±0,11 p <0,0005 |
| L5 | 295±25 p <0,05 | 3,7±2 N.S. |
| H5 | 272±41 p <0,05 | 0,5±0,10 p <0,0005 |

Les résultats obtenus sont considérés comme significatifs pour un $p < 0,05$, c'est-à-dire lorsque le risque que les résultats chiffrés qui pourraient être obtenus en multipliant les essais du même type ne soient pas contenus dans les intervalles indiqués, est inférieur à 5% des cas (test de Student décrit par D. Schwartz dans l'ouvrage intitulé «Méthodes statistiques à l'usage des médecins et des biologistes», édité par Flammarion Médecine Sciences, 1963) (N.S. non significatif). Dans ces conditions, on constate que les animaux ainsi traités présentent une résistance augmentée envers le carcinome de Lewis.

Ce test représente à ce jour l'un des tests les plus sévères qui soient. Le carcinome de Lewis constitue en effet la tumeur solide murine considérée comme la plus maligne parmi les tumeurs connues chez l'animal. Les résultats indiqués ci-après présentent par conséquent un degré de présomption considéré à ce jour comme l'un des plus significatifs à l'égard de l'efficacité que l'on est en droit d'attendre de ces substances chez des animaux supérieurs et chez l'homme; cette présomption étant ici encore renforcée par le fait que les régressions observées des tumeurs, surtout avec les composés «1-5» et «H5», ont été obtenues grace à une administration des principes actifs postérieurement à l'inoculation des tumeurs.

Les expérimentations dont les résultats ont été exposés ci-dessus font donc apparaître que les peptides selon l'invention possèdent des propriétés immunostimulantes non spécifiques, puisqu'ils sont, après injection à la souris, capables d'augmenter leur résistance envers des organismes aussi différents que les bactéries très virulentes pour la souris: L. monocytogenes, S. typhimurium et les tumeurs murines les plus malignes, dont le carcinome de Lewis est représentatif.

L'effet immunostimulant est obtenu douze heures avant l'injection des bactéries et vingt-quatre heures après l'injection des cellules malignes.

On remarque l'activité particulièrement importante des pentapeptides testés, plus particulièrement de ceux dans lesquels le résidu aminoacyle N-terminal est constitué par la lysine et – en ce qui concerne l'activité antitumorale – surtout l'histidine.

Ces différents composés peuvent donc non seulement être utilisés comme réactifs de laboratoire, notamment à titre de références ou d'étalons pour étudier la présence ou non de propriétés immunostimulantes non spécifiques dans des substances étudiées sous cet angle, mais encore comme principes actifs de médicaments, susceptibles d'exercer une stimulation du système réticulo-endothelial.

Il est intéressant, en ce qui concerne plus particulièrement le pentapeptide 1-5, de noter que celui-ci ne doit normalement pas entraîner d'accident de sensibilisation chez l'homme, puisqu'il peut, à la limite, être considéré comme un produit de dégradation de la bradykinine chez le mammifère. Il est au demeurant totalement inactif à l'égard de l'iléon de cobaye, et d'une façon générale dépourvu de toutes les propriétés antérieurement connues de la bradykinine.

Les peptides selon l'invention peuvent constituer le principe actif de médicaments stimulants du système réticulo-endothélial indiqués notamment pour le traitement ou la prévention chez l'homme et l'animal des maladies mettant en jeu une activation des macrophages, telles que les maladies infectieuses causées par des germes bactériens et, par exemple, des infections du type tuberculose, pasteurelloses, brucelloses, listerioses ou infections à bactéries gram négatif ou des maladies causées par certains virus tels que le virus de la grippe.

Ils peuvent également être utilisés pour le traitement de maladies dues à certains parasites présentant au cours de leur cycle une phase de passage dans la circulation sanguine ou lymphatique, par exemple le paludisme, la bilharziose et la filariose. Ils peuvent encore être utilisés pour le traitement des toxi-infections, c'est-à-dire d'infections dues à des agents microbiens dont les actions ne s'exercent pas seulement à un niveau localisé, ou même à un niveau général, mais aussi à des agents induisant la libération de toxines capables d'agir à distance. Ils sont applicables à la prévention d'infections post-chirurgicales de toutes natures. Enfin, leur action à l'égard du carcinome de Lewis les rend, avec un grand degré de présomption, aptes à freiner la prolifération de cellules susceptibles de former des tumeurs solides, voire à en assurer l'éradication.

Les compositions contenant de tels peptides peuvent être administrées par voie intraveineuse, intramusculaire ou sous-cutanée, en solution dans des véhicules pharmaceutiquement acceptables et stériles, telles que des solutions de sérum physiologique (solution saline ou sérum glucosé). Les quantités administrées devront être établies par le clinicien. Ces quantités seront avantageusement dosées de façon telle que les concentrations sanguines, notamment au moment de l'injection si c'est cette voie dont l'adoption est envisagée, atteignent une valeur de $10^{-5}$ à $10^{-3}$ molaire, plus particulièrement de $0,25.10^{-4}$ à $1.10^{-4}$ molaire.

Les agents actifs selon l'invention peuvent également être administrés par voie orale, lorsqu'ils sont associés à des excipients solides ou liquides pharmaceutiquement acceptables. Leur administration peut également être envisagée par voie rectale, lorsqu'ils sont associés à des excipients appropriés à ce mode d'administration. Ils peuvent également être administrés par voie externe, par exemple sous forme d'aérosol avec un véhicule approprié à ce mode d'administration, par exemple pour le traitement d'infections nasales.

Les agents selon l'invention peuvent également être utilisés comme principes actifs de pommades, notamment lorsqu'ils sont appliqués à des traitmenets de cicatrisation externe. Ces pommades peuvent être réalisées de toutes façons en soi connues en ayant recours à des excipients pharmaceutiques de pommades, par exemple de la lanoline. La concentration en principe actif dans ces pommades est normalement comprise entre 0,5 et 2% en poids.

Il va de soi que les indications de doses qui précèdent n'ont d'autre but que d'illustrer les possibilités d'utilisation de l'invention et qu'en fait la détermination de ces doses relèvera toujours de l'appréciation du clinicien.

**Revendications**

1. Composition contenant, en association avec un véhicule pharmceutiqement acceptable, un peptide ou l'un de ses dérivés fonctionnels, pour la mise en œuvre de traitements thérapeutiques mettant en jeu les propriétés biologiques, notamment immunostimulantes, de ce peptide ou dérivé, caractérisée en ce que ce peptide comporte au plus sept résidus aminoacyle et est caractérisé en ce qu'il contient lui-même une séquence prolyl → prolyl → glycyle, le groupe carboxylique du résidu glycyle étant, le cas échéant, engagé dans une liaison peptidique avec un autre résidu aminoacyle.

2. Composition selon la revendication 1, caractérisé en ce que ledit peptide contient une séquence prolyl → prolyl → glycyl → phénylalanyle.

3. Composition selon la revendication 2, caractérisée en ce que le résidu phénylalanyle porte l'extrémité C-terminale dudit peptide.

4. Composition selon l'une quelconque des revendications 1 à 3, caractérisée en ce que le groupe aminé du premier résidu prolyle de la susdite séquence prolyl → prolyl → glycyle est engagé dans une liaison peptidique avec un résidu arginyle, lysyle ou histidyle.

5. Composition selon l'une quelconque des revendictions 1 à 4, caractérisé en ce qu'elle contient un pentapeptide tel que l'un de ceux qui possèdent les formules suivantes:
Arginyl → prolyl → prolyl → glycyl → phénylalanine;
Lysyl → prolyl → prolyl → glycyl → phénylalanine;
Histidyl → prolyl → prolyl → glycyl → phénylalanine.

6. Composition selon l'une quelconque des revendictions 1 à 5, caractérisée en ce que tous les résidus aminoacyle dudit peptide, à l'exception de la glycine, sont lévogyres.

7. Peptide comportant au plus sept résidus aminoacyle et contenant une séquence prolyl → prolyl → glycyle, le groupe carboxylique du résidu glycyle étant, le cas échéant, engagé dans une liaison peptidique avec un autre résidu aminoacyle, caractérisé en ce que le groupe aminé du premier résidu prolyle de la susdite séquence prolyl → prolyl → glycyle est engagé dans une liaison peptidique avec un résidu aminoacyle distinct de l'arginyle, de préférence du lysyle ou de l'histidyle.

8. Peptide selon la revendication 7 de formule: Lysyl → prolyl → prolyl → glycyl → phénylalanyle.

9. Peptide selon la revendication 7, de formule: Histidyl → prolyl → prolyl → glycyl → phénylalanyle.

10. Peptide selon l'une quelconque des revendications 7 à 9, caractérisé en ce que, à l'exception de la glycine, tous les résidus aminoacyle dont il est formé sont lévogyres.

11. Les dérivés fonctionnels d'un peptide selon l'une quelconque des revendications 7 à 10.

## Patentansprüche

1. Zusammensetzung, die zusammen mit einem pharmazeutisch annehmbaren Träger ein Peptid oder eines seiner funktionellen Derivate enthält, zur therapeutischen Behandlung aufgrund der biologischen, insbesonderen immunostimulierenden Eigenschaften des Peptids oder Derivates, dadurch gekennzeichnet, dass das Peptid höchstens sieben Aminoacylreste enthält und dadurch charakterisiert ist, dass es eine Sequenz Prolyl → Prolyl → Glycyl enthält, wobei die Carboxylgruppe des Glycylrestes gegebenenfalls eine Peptidbindung mit einem anderen Aminoacylrest bildet.

2. Zusammensetzung nach Anspruch 1, dadurch gekennzeichnet, dass das Peptid eine Sequenz Prolyl → Prolyl → Glycyl → Phenylalanyl enthält.

3. Zusammensetzung nach Anspruch 2, dadurch gekennzeichnet, dass der Phenylalanylrest das äusserste C-terminale Ende des Peptids trägt.

4. Zusammensetzung nach einem der Ansprüche 1 bis 3, dadurch gekennzeichnet, dass die Aminogruppe des ersten Prolylrestes der Sequenz Prolyl → Prolyl → Glycyl mit einem Arginyl-, Lysyl- oder Histidyl-rest eine Peptidbindung bildet.

5. Zusammensetzung nach einem der Ansprüche 1 bis 4, dadurch gekennzeichnet, dass sie ein Pentapeptid mit einer der folgenden Formeln enthält:
Arginyl → Prolyl → Prolyl → Glycyl → Phenylalanin;
Lysyl → Prolyl → Prolyl → Glycyl → Phenylalanin;
Histidyl → Prolyl → Prolyl → Glycyl → Phenylalanin.

6. Zusammensetzung nach einem der Ansprüche 1 bis 5, dadurch gekennzeichnet, das alle Aminoacylreste des Peptids mit der Ausnahme von Glycin der L-Reihe (lévogyres) angehören.

7. Peptid mit höchstens sieben Aminoacylresten und welches eine Sequenz Prolyl → Prolyl → Glycyl enthält, wobei die Carboxylgruppe des Glycylrestes gegebenenfalls eine Peptidbindung mit einem anderen Aminoacylrest bildet, dadurch gekennzeichnet, dass die Aminogruppe des ersten Prolylrestes der Sequenz Prolyl → Prolyl → Glycyl mit einem Aminoacylrest, der Arginyl, und vorzugsweise Lysyl oder Histidyl ist, eine Peptidbindung bildet.

8. Peptid nach Anspruch 7 der Formel: Lysyl → Prolyl → Prolyl → Glycyl → Phenylalanyl.

9. Peptid nach Anspruch 7 der Formel: Histidyl → Prolyl → Prolyl → Glycyl → Phenylalanyl.

10. Peptid nach einem der Ansprüche 7 bis 9, dadurch gekennzeichnet, dass alle Aminoacylreste mit Ausnahme von Glycin der L-Reihe (lévogyres) angehören.

11. Die funktionellen Derivate eines Peptids nach einem der Ansprüche 7 bis 10.

## Claims

1. Composition containing in association with a pharmaceutically acceptable vehicle, a peptide or one of its functional derivatives, for therapeutical treatments, involving the biological, particularly immunostimulating, properties of this peptide or derivative, characterized by the fact that this peptide comprises at most 7 amino-acyl residues and is characterized by the fact that it contains itself a prolyl → prolyl → glycyl sequence, the carboxylic group of the glycyl residue being possibly engaged in a peptidic bond with another amino-acyl residue.

2. Composition according to claim 1, characterized by the fact that said peptide contains a prolyl → prolyl → glycyl → phenylalanyl sequence.

3. Composition according to claim 2, characterized by the fact that the phenylalanyl residue bears the C-terminal end of said peptide.

4. Composition according to anyone of claims 1 to 3, characterized by the fact that the amino group of the first prolyl residue of said prolyl → prolyl → glycyl sequence is engaged in a peptidic bond with an arginyl, lysyl or histidyl residue.

5. Compositon according to anyone of claims 1 to 4, characterized by the fact that it contains a pentapeptide such as the one which presents the following formula:
arginyl → prolyl → prolyl → glycyl → phenylalanine;
lysyl → prolyl → prolyl → glycyl → phenylalanine;
histidyl → prolyl → prolyl → glycyl → phenylalanine.

6. Composition according to anyone of claims 1 to 5, characterized by the fact that all the amino-acyl residues of said peptide, except glycine, are levorotatory.

7. Peptide comprising at most 7 amino-acyl residues containing a prolyl → prolyl → glycyl sequence, the carboxylic group of the glycyl residue being optionally engaged in a peptidic bond with another amino-acyl residue, characterized by the fact that the amino group of the first prolyl residue of said prolyl → prolyl → glycyl sequence

is engaged in a peptidic bond with an amino-acyl residue different from arginyl residue, preferably lysyl or histidyl.

8. Peptide according to claim 7 of the formula: lysyl → prolyl → prolyl → glycyl → phenylalanyl.

9. Peptide according to claim 7 of the formula: histidyl → prolyl → prolyl → glycyl → phenylalanyl.

10. Peptide according to claims 7 to 9, characterized by the fact that, except glycine, all the amino-acyl residues by which it is constituted, are levorotatory.

11. Functional derivatives of a peptide according to anyone of claims 7 to 10.